# EUROPEAN PATENT APPLICATION

(11) **EP 4 834 348 A1**
(43) Date of publication of application: **04.11.2026**
(21) Application number: 24911497.6
(22) Date of filing: 27.12.2024
(51) Int. Cl.: A61K 31/519, A61K 9/20, A61K 9/48, A61K 9/14

(54) **PHARMACEUTICAL COMPOSITION**

(30) Priority: 29.12.2023 CN 202311864682
(71) Applicant: Prime Gene Therapeutics Co., Ltd., Beijing 100176 (CN)
(72) Inventor: WANG, Zaiquan, Beijing 100176 (CN); LI, Baocheng, Beijing 100176 (CN); LI, Ming, Beijing 100176 (CN); LIU, Yan, Beijing 100176 (CN); ZHANG, Yaqi, Beijing 100176 (CN); WANG, Yanwei, Beijing 100176 (CN); WU, Mangang, Beijing 100176 (CN); ZHU, Li, Beijing 100176 (CN); YE, Dong, Beijing 100176 (CN); DU, Daniel Yunlong, Beijing 100176 (CN)
(74) Representative: Winter, Brandl - Partnerschaft mbB
(86) International application number: PCT/CN2024/143247
(87) International publication number: WO 2025/140579

(57) **Abstract**

The present application relates to a pharmaceutical composition comprising an active ingredient Compound 1, a pharmaceutically acceptable polymeric carrier, a surfactant, and other pharmaceutically acceptable excipients; wherein said active ingredient, said polymeric carrier, and optionally at least a portion of said surfactant are present in the form of a solid dispersion. The present application discloses for the first time a pharmaceutical composition comprising Compound 1, which has stable product quality and good bioavailability. Based on the property of Compound 1 as a JAK inhibitor, the composition has broad application prospects for alopecia areata, vitiligo, atopic dermatitis, psoriasis, membranous nephropathy, ankylosing spondylitis, peripheral T-cell lymphoma, ulcerative colitis, rheumatoid arthritis, systemic lupus erythematosus, Crohn's disease, etc.

## Description

### Technical Field

The present invention belongs to the field of pharmaceutical technology, and specifically relates to a pharmaceutical composition comprising 2-[3-[3-amino-4-(7H-pyrrolo[2,3-d]pyrimidin-4-yl)-1H-pyrazol-1-yl]-1-[1-[3-fluoro-2-(trifluoromethyl)isonicotinoyl]piperidin-4-yl] azetidin-3-yl]acetonitrile.

### Background Art

2-[3-[3-amino-4-(7H-pyrrolo[2,3-d]pyrimidin-4-yl)-1H-pyrazol-1-yl]-1-[1-[3-fluoro-2-(trifluoromethyl)isonicotinoyl] piperidin-4-yl]azetidin-3-yl]acetonitrile (hereinafter referred to as "Compound 1") is a JAK inhibitor having the following structural formula:

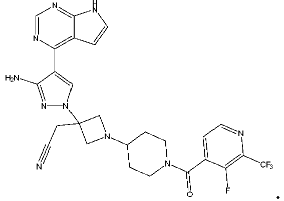

Compound 1 has low solubility in aqueous media (i.e., solubility in water at 25°C is less than 1 mg/mL), poor permeability and low oral bioavailability. In order to improve its bioavailability and enhance druggability, many solubilization techniques have been attempted, such as micronization of active pharmaceutical ingredients, studies on salt formation or crystalline form, microemulsion/self-microemulsion, surfactant solubilization, cyclodextrin inclusion, solid dispersion, co-crystallization and the like. There is a need in the art for a pharmaceutical composition of this compound with high bioavailability.

### Summary

It has been surprisingly found that a pharmaceutical composition comprising a solid dispersion can significantly improve the bioavailability of Compound 1.

In one aspect, the present application provides a solid dispersion, which comprises:
an active ingredient, which is 2-[3-[3-amino-4-(7H-pyrrolo[2,3-d]pyrimidin-4-yl)-1H-pyrazol-1-yl]-1-[1-[3-fluoro-2-(trifluoromethyl)isonicotinoyl]piperidin-4-yl]azetidin-3-yl] acetonitrile having the following structural formula, or a pharmaceutically acceptable salt thereof, 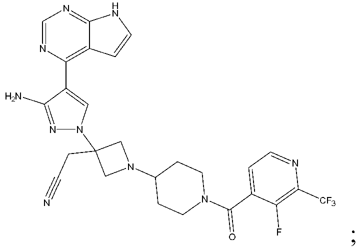
a pharmaceutically acceptable polymeric carrier; and
a surfactant;
wherein said surfactant is selected from sodium lauryl sulfate, vitamin E polyethylene glycol succinate, poloxamer, polyethylene glycol 15-hydroxystearate, sodium stearyl fumarate, sodium docusate, cetrimide, benzethonium chloride, cetylpyridinium chloride, lauric acid, polyoxyethylene alkyl ethers, polyoxyethylene sorbitan fatty acid esters; polyoxyethylene castor oil derivatives, polyoxyethylene stearates, poloxamers, polysorbates, sorbitan fatty acid esters; preferably sodium lauryl sulfate.

The present application also provides a pharmaceutical composition, which comprises:
an active ingredient, which is 2-[3-[3-amino-4-(7H-pyrrolo[2,3-d]pyrimidin-4-yl)-1H-pyrazol-1-yl]-1-[1-[3-fluoro-2-(trifluoromethyl)isonicotinoyl]piperidin-4-yl]azetidin-3-yl] acetonitrile having the following structural formula, or a pharmaceutically acceptable salt thereof, 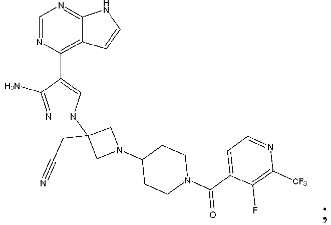
a pharmaceutically acceptable polymeric carrier;
a surfactant; and
other pharmaceutically acceptable excipients;
wherein said surfactant is selected from sodium lauryl sulfate, vitamin E polyethylene glycol succinate, poloxamer, polyethylene glycol 15-hydroxystearate, sodium stearyl fumarate, sodium docusate, cetrimide, benzethonium chloride, cetylpyridinium chloride, lauric acid, polyoxyethylene alkyl ethers, polyoxyethylene sorbitan fatty acid esters; polyoxyethylene castor oil derivatives, polyoxyethylene stearates, poloxamers, polysorbates, and sorbitan fatty acid esters, preferably sodium lauryl sulfate;
wherein said active ingredient, polymeric carrier and optionally at least a portion of the surfactant are present in the form of a solid dispersion.

In one embodiment, said polymeric carrier is one or more selected from copovidone, hydroxypropyl methylcellulose phthalate (HPMCP), hydroxypropyl methylcellulose acetate succinate (HPMCAS), hydroxypropyl methylcellulose (HPMC), polymethacrylates, hydroxypropyl cellulose (HPC) and cellulose acetate phthalate (CAP); preferably, said polymeric carrier is one or more selected from copovidone, hydroxypropyl methylcellulose phthalate (HPMCP), hydroxypropyl methylcellulose acetate succinate (HPMCAS) and hydroxypropyl methylcellulose (HPMC); more preferably, said polymeric carrier is copovidone.

In one embodiment, in said solid dispersion, the weight ratio of said active ingredient to said polymeric carrier is from 1:1 to 1:20, preferably from 1:2 to 1:10, and the weight ratio of said active ingredient to said surfactant is from 1:0.001 to 1:0.5.

In one embodiment, based on the total weight of said pharmaceutical composition,
the active ingredient accounts for 0.1 wt% to 30 wt%;
the polymeric carrier accounts for 1.0 wt% to 90.0 wt%;
the surfactant accounts for 0.1 wt% to 10.0 wt%;
the total weight of other pharmaceutically acceptable excipients accounts for 10 wt% to 98 wt%.

In one embodiment, the other pharmaceutically acceptable excipients include one or more of a filler, a disintegrant, a glidant, a lubricant and a binder.

In one embodiment, the filler is one or more selected from lactose, sugars, starch, modified starch, mannitol, sorbitol, inorganic salts, cellulose derivatives (e.g., microcrystalline cellulose, cellulose) and xylitol; preferably, the filler is one or more selected from microcrystalline cellulose, mannitol, sorbitol and lactose.

In one embodiment, the filler is microcrystalline cellulose or mannitol, or a combination of microcrystalline cellulose and mannitol;
when present, the content of microcrystalline cellulose is from 0 wt% to 99.0 wt%, based on the total weight of said pharmaceutical composition; and/or
when present, the content of mannitol is from 0 wt% to 99.0 wt%, based on the total weight of said pharmaceutical composition.

In one embodiment, the disintegrant is one or more selected from croscarmellose sodium, crospovidone, polyvinylpyrrolidone, sodium starch glycolate, corn starch, microcrystalline cellulose, hydroxypropyl methylcellulose and hydroxypropyl cellulose; preferably, the disintegrant is one or more selected from croscarmellose sodium, crospovidone, polyvinylpyrrolidone and microcrystalline cellulose.

In one embodiment, the disintegrant is croscarmellose sodium;
when present, the content of croscarmellose sodium is from 0.5 wt% to 20.0 wt%, based on the total weight of said pharmaceutical composition.

In one embodiment, the glidant is one or two selected from silicon dioxide and talc powders; preferably, the glidant is silicon dioxide;
when present, the content of silicon dioxide is from 0.1 wt% to 10.0 wt%, based on the total weight of said pharmaceutical composition.

In one embodiment, the lubricant is one or more selected from magnesium stearate, magnesium lauryl stearate, sodium stearyl fumarate, stearic acid, calcium stearate, zinc stearate, potassium benzoate, sodium benzoate, myristic acid, palmitic acid, mineral oil, hydrogenated castor oil, medium-chain triglycerides, poloxamers, polyethylene glycols and talc powders; preferably, the lubricant is one or two selected from magnesium stearate and sodium stearyl fumarate.

In one embodiment, the lubricant is magnesium stearate;
when present, the content of magnesium stearate is from 0.05 wt% to 2.0 wt%, based on the total weight of said pharmaceutical composition.

In one embodiment, said pharmaceutical composition comprises, based on the total weight of said pharmaceutical composition, the following components:
0.1 wt% to 30.0 wt% of an active ingredient;
1.0 wt% to 90.0 wt% of copovidone;
0 wt% to 99.0 wt% of mannitol;
0 wt% to 99.0 wt% of microcrystalline cellulose;
0.5 wt% to 20.0 wt% of croscarmellose sodium;
0.1 wt% to 10.0 wt% of sodium lauryl sulfate;
0.1 wt% to 10.0 wt% of silicon dioxide;
0.05 wt% to 2.0 wt% of magnesium stearate;
wherein, said active ingredient, copovidone and optionally at least a portion of the sodium lauryl sulfate are present in the form of a solid dispersion.

In one embodiment, said solid dispersion is prepared by spray drying process or hot-melt extrusion process; preferably, said solid dispersion is prepared by spray drying process.

In one embodiment, the pharmaceutical composition is an oral solid preparation, including a tablet, a granule, a powder, a dry suspension, or a capsule.

In one embodiment, said pharmaceutical composition is a tablet.

The present application discloses for the first time a pharmaceutical composition comprising Compound 1, which has stable product quality and good bioavailability.

### Description of the Drawings

Figure 1 shows the effect of dosage form on the pharmacokinetics of Compound 1;
Figure 2 shows a comparison of the dissolution of Compound 1 in different types of preparation;
Figure 3 shows a comparison of the effect of fillers on the dissolution of Compound 1;
Figure 4 shows the effect of the way of glidant addition on the dissolution of Compound 1;
Figure 5 shows the effect of the amount of glidants on the dissolution of Compound 1;
Figure 6 shows the effect of the amount of lubricants on the dissolution of Compound 1;
Figure 7 shows the effect of the amount of disintegrants on the dissolution of Compound 1;
Figure 8 shows the effect of the amount of surfactant on the dissolution of Compound 1;
Figure 9 shows the effect of product hardness on the dissolution of Compound 1;
Figure 10 shows the effect of coating on the dissolution of Compound 1;
Figure 11 shows the dissolution profile of Compound 1 in stability tests;
Figure 12 shows results of the crystal form test of Compound 1 in stability tests.

### Detailed Description

The present application is further described in detail below with reference to the drawings and examples. Through these descriptions, the features and advantages of the present application will become clearer and more apparent.

The term "exemplary" used herein means "serving as an example, embodiment, or illustration". Any embodiment described herein as "exemplary" is not necessarily to be construed as preferred or advantageous over other embodiments. Although various aspects of the embodiments are shown in the drawings, the drawings are not necessarily drawn to scale unless otherwise indicated.

Furthermore, the technical features involved in different embodiments of the present application described below may be combined with each other as long as they do not conflict with each other.

The term "solid dispersion" as used herein refers to a system in which an active ingredient is dispersed in an excipient carrier. In a solid dispersion, one or more active ingredients are highly uniformly dispersed in an inert excipient or framework in the state of a molecular, amorphous, or microcrystalline, forming a dispersed system. As for the drug state within this system, the solid dispersion may, in this sense, include compositions in which the drug is dispersed in the excipient carrier in discrete states as crystalline or amorphous drug, or as individual molecules. As for the entire drug excipient complex, the solid dispersion may be a relatively large solid substance, such as pellets, tablets, films, or strips; or it may be present as a free-flowing powder composed of micron-scale or nano-scale primary particles or aggregates thereof. The final state of the solid dispersion composition mainly depends on the processing technique.

Methods for preparing the solid dispersion are known in the art and generally include the steps of dissolving this drug and this polymer in a common solvent and evaporating this solvent. This solvent can be selected in a conventional manner according to the properties of the polymer used. Commonly solvents are acetone, acetone/dichloromethane, methanol/dichloromethane, acetone/water, acetone/methanol, acetone/ethanol, dichloromethane/ethanol, or ethanol/water. Methods for evaporating the solvent include rotary evaporation, spray drying, freeze drying and thin film evaporation. Traditional methods for preparing the solid dispersion include the melting process and the solvent process. In recent years, the hot-melt extrusion process (melting process), as a new method for preparing the solid dispersion, has attracted much attention from pharmaceutical researchers at home and abroad. This method achieves the transfer, conveying, shearing, mixing and melt extrusion of materials in a single-screw or double-screw extruder heated section by section. Compared with traditional preparation methods, the hot-melt extrusion process has the characteristics of high production efficiency, no need for organic solvents and suitability for industrial production, and the like. However, for drugs and carriers with high melting points, the melting process and hot-melt extrusion process are prone to thermal decomposition of the drug and carrier, thus limiting the widespread application of this method. The spray drying process (solvent process) involves dissolving the drug and carrier material together in an organic solvent and then removing the organic solvent to obtain a uniformly dispersed dispersion. Compared with hot-melt extrusion, the spray drying process is more suitable for drugs with poor thermal stability and has the characteristics of short development cycles and being conducive to batch scale-up.

In one aspect, the present application provides a solid dispersion, which comprises:
an active ingredient, which is 2-[3-[3-amino-4-(7H-pyrrolo[2,3-d]pyrimidin-4-yl)-1H-pyrazol-1-yl]-1-[1-[3-fluoro-2-(trifluoromethyl)isonicotinoyl]piperidin-4-yl]azetidin-3-yl] acetonitrile having the following structural formula, or a pharmaceutically acceptable salt thereof, 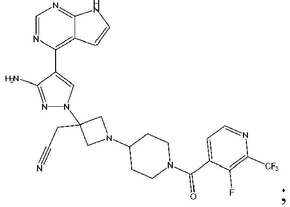
a pharmaceutically acceptable polymeric carrier, and
a surfactant;
wherein, said surfactant is selected from sodium lauryl sulfate, vitamin E polyethylene glycol succinate, polyethylene glycol 15-hydroxystearate, sodium stearyl fumarate, sodium docusate, cetrimide, benzethonium chloride, cetylpyridinium chloride, lauric acid, polyoxyethylene alkyl ethers, polyoxyethylene sorbitan fatty acid esters; polyoxyethylene castor oil derivatives, polyoxyethylene stearates, poloxamers, polysorbates, sorbitan fatty acid esters, preferably sodium lauryl sulfate.

Compound 1 is a JAK inhibitor that can be used for the treatment or prevention of various autoimmune diseases including atopic dermatitis, prurigo nodularis, vitiligo, scleroderma, psoriasis, membranous nephropathy, ankylosing spondylitis, peripheral T-cell lymphoma, ulcerative colitis, rheumatoid arthritis, systemic lupus erythematosus, Crohn's disease and alopecia areata, and the like. It is disclosed in CN109867676B, the entire content of which is incorporated herein by reference.

As shown in Example 1 below, the solubility of Compound 1 in media of different pH is clearly pH-dependent, with higher solubility in an acidic environment and almost insoluble in neutral, alkaline environments and water. Among the common solvents used for preparing the solid dispersion by spray drying, acetone has a relatively high solubility for Compound 1.

In one embodiment, said solid dispersion comprises at least 5 mg of Compound 1, for example, 5 mg of Compound 1, 15 mg of Compound 1, 45 mg of Compound 1, 75 mg of Compound 1, 100 mg of Compound 1, 150 mg of Compound 1, or 250 mg of Compound 1.

In one embodiment, said solid dispersion comprises up to 5 mg of Compound 1, for example, said solid dispersion comprises 0.5 mg, 0.75 mg, 1 mg, 2 mg, 3 mg, 4 mg, or 5 mg of Compound 1.

The solid dispersion of the present application comprises a pharmaceutically acceptable polymeric carrier. In one embodiment, the polymeric carrier is one or more selected from copovidone, hydroxypropyl methylcellulose phthalate (HPMCP), hydroxypropyl methylcellulose acetate succinate (HPMCAS), hydroxypropyl methylcellulose (HPMC), polymethacrylates, hydroxypropyl cellulose (HPC) and cellulose acetate phthalate (CAP), and combinations of one or more thereof can be used. Preferably, said polymeric carrier is one or more selected from copovidone, hydroxypropyl methylcellulose phthalate (HPMCP), hydroxypropyl methylcellulose acetate succinate (HPMCAS) and hydroxypropyl methylcellulose (HPMC). In one embodiment, in said solid dispersion, the weight ratio of said active ingredient to said polymeric carrier is from 1:1 to 1:20, for example, from 1:1 to 1:15, preferably from 1:2 to 1:10.

In the present application, said polymeric carrier can effectively increase the solubility of Compound 1 in pH 6.8 phosphate buffer and effectively maintain the supersaturated state of Compound 1 in the medium. In particular, copovidone has a good solubilizing effect on Compound 1 and a good ability to maintain supersaturated state, and is therefore more preferred.

The solid dispersion of the present application further comprises a pharmaceutically acceptable surfactant. This surfactant is selected from sodium lauryl sulfate, vitamin E polyethylene glycol succinate, polyethylene glycol 15-hydroxystearate, sodium stearyl fumarate, sodium docusate, cetrimide, benzethonium chloride, cetylpyridinium chloride, lauric acid, polyoxyethylene alkyl ethers, polyoxyethylene sorbitan fatty acid esters; polyoxyethylene castor oil derivatives, polyoxyethylene stearates, poloxamers, polysorbates, sorbitan fatty acid esters, preferably sodium lauryl sulfate (SDS). The inventors of the present application unexpectedly found that, compared with different surfactants, SDS has a very clear solubilizing ability for Compound 1, can very effectively increase the solubility of Compound 1, and can maintain its supersaturated state for a long time. In one embodiment, the weight ratio of said active ingredient to said surfactant is from 1:0.001 to 1:0.5. Within the above range, the solubilizing ability of the surfactant for Compound 1 and the ability to maintain supersaturated state can achieve a better balance.

The solid dispersion of the present application can be used as one of the components for preparing subsequent pharmaceutical compositions. Moreover, the inventors of the present application also found that, when combined with other excipients such as a filler, a disintegrant, a glidant, a lubricant and a binder, and the like to prepare a pharmaceutical composition, it cannot achieve the effect of solubilization if the individual components of the solid dispersion, particularly the active ingredient and the polymeric carrier, are simply mixed with these other excipients to prepare the pharmaceutical composition. It is required to firstly make the active ingredient and the polymeric carrier into a solid dispersion, and then combine them with other excipients such as a filler, a disintegrant, a glidant, a lubricant and a binder and the like to prepare the pharmaceutical composition. This can achieve the solubilizing effect well.

Methods for preparing the solid dispersion are known in the art and generally include the steps of dissolving this drug and this polymer in a common solvent and evaporating this solvent. This solvent can be selected in a conventional manner according to the properties of the polymer used. Common solvents are acetone, acetone/dichloromethane, methanol/dichloromethane, acetone/water, acetone/methanol, acetone/ethanol, dichloromethane/ethanol, or ethanol/water. Methods for evaporating the solvent include rotary evaporation, spray drying, freeze drying and thin film evaporation. In the present application, the solid dispersion sample comprising Compound 1 and the polymer can be prepared by a spray drying process: dissolving Compound 1 and the polymeric carrier in a low-boiling-point solvent, and using a spray dryer to prepare the solid dispersion sample comprising Compound 1 and the polymer. Based on the solubility of Compound 1 and the polymeric carrier in different solvents, acetone can be used as the solvent.

For example, when the polymeric carrier is copovidone, Compound 1, copovidone and SDS are dissolved in acetone, and the acetone solution is spray-dried using a spray dryer at an inlet air temperature of 100°C and an outlet air temperature of 50°C to obtain the solid dispersion of the present application.

In a second aspect, the present application provides a pharmaceutical composition, which comprises:
an active ingredient, which is 2-[3-[3-amino-4-(7H-pyrrolo[2,3-d]pyrimidin-4-yl)-1H-pyrazol-1-yl]-1-[1-[3-fluoro-2-(trifluoromethyl)isonicotinoyl]piperidin-4-yl]azetidin-3-yl] acetonitrile having the following structural formula, or a pharmaceutically acceptable salt thereof, 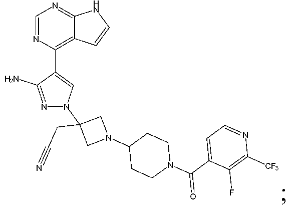
a pharmaceutically acceptable polymeric carrier;
a surfactant; and
other pharmaceutically acceptable excipients;
wherein said surfactant is selected from sodium lauryl sulfate, vitamin E polyethylene glycol succinate, polyethylene glycol 15-hydroxystearate, sodium stearyl fumarate, sodium docusate, cetrimide, benzethonium chloride, cetylpyridinium chloride, lauric acid, polyoxyethylene alkyl ethers, polyoxyethylene sorbitan fatty acid esters; polyoxyethylene castor oil derivatives, polyoxyethylene stearates, poloxamers, polysorbates, sorbitan fatty acid esters, preferably sodium lauryl sulfate;
wherein said active ingredient, the polymeric carrier and optionally at least a portion of the surfactant are present in the form of a solid dispersion.

In the pharmaceutical composition of the present application, it is required to firstly make the active ingredient and the polymeric carrier into a solid dispersion, and then combine them in the form of solid dispersion with other excipients to further prepare the pharmaceutical composition.

In terms of the surfactant SDS, it can be firstly made into a solid dispersion together with the active ingredient and the polymeric carrier, and then combined in the form of solid dispersion with other excipients to further prepare the pharmaceutical composition. Alternatively, after the active ingredient and the polymeric carrier are made into a solid dispersion, the surfactant can be combined with this solid dispersion together with other excipients to prepare the pharmaceutical composition. The inventors of the present application found that the surfactant can also very effectively increase the solubility of Compound 1 and maintain its supersaturated state for a long time by using the latter approach. Of course, when preparing the pharmaceutical composition, a portion of the surfactant can also be firstly made into a solid dispersion together with the active ingredient and the polymeric carrier, and then another portion of the surfactant is combined with this solid dispersion together with other excipients. This is also within the protection scope of the present application. The ratio of these two portions of surfactant can be selected as needed. The surfactant that is made into the solid dispersion together with the active ingredient and the polymeric carrier can account for 0 wt% to 100 wt% of the total amount of surfactant, for example, 30 wt% to 100 wt%, or 60 wt% to 100 wt%, or 70 wt% to 100 wt%.

In this pharmaceutical composition, the selection of the polymeric carrier and its ratio to the active ingredient can refer to the solid dispersion section aforementioned in the present application, and the relevant description applies equally to the pharmaceutical composition section of the present application, and will not be repeated here.

In one embodiment, based on the total weight of said pharmaceutical composition,
the active ingredient accounts for 0.1 wt% to 30 wt%;
the polymeric carrier accounts for 1.0 wt% to 90.0 wt%;
the surfactant accounts for 0.1 wt% to 10.0 wt%.

The pharmaceutical composition of the present application further comprises other pharmaceutically acceptable excipients, the total weight of which accounts for 10 wt% to 98 wt%, based on the total weight of said pharmaceutical composition.

In the pharmaceutical composition of the present application, the amounts of the polymeric carrier and other pharmaceutically acceptable excipients can vary widely and can be adjusted according to the content and requirements of the active pharmaceutical ingredient (API). For example, the polymeric carrier can account for 1.0 wt% to 90.0 wt%, based on the total weight of said pharmaceutical composition, for example, less than or equal to 85 wt%, less than or equal to 80 wt%, less than or equal to 75 wt%, or less than or equal to 70 wt%.

The other pharmaceutically acceptable excipients can be various excipients commonly used in pharmaceuticals, such as one or more of a filler, a disintegrant, a glidant, a lubricant and a binder and the like, and the total amount thereof can be the sum of the amounts of other pharmaceutical excipients excluding the polymeric carrier and the surfactant. For example, the other pharmaceutically acceptable excipients account for 10 wt% to 98 wt%, based on the total weight of said pharmaceutical composition, for example, 12 wt% to 98 wt%.

The pharmaceutical composition in the present invention may comprise a filler. In one embodiment, said filler can be one or more selected from lactose, sugars, starch, modified starch, mannitol, sorbitol, inorganic salts, cellulose derivatives (e.g., microcrystalline cellulose, cellulose), calcium sulfate, xylitol, and the combination thereof. Preferably, one or more of microcrystalline cellulose, mannitol, sorbitol, lactose and the combination thereof can be used.

In one embodiment, said filler can be microcrystalline cellulose or mannitol, or a combination of microcrystalline cellulose and mannitol.

When present, the amount of microcrystalline cellulose is from 0 wt% to 99.0 wt%, based on the total weight of said pharmaceutical composition; and/or
when present, the amount of mannitol is from 0 wt% to 99.0 wt%, based on the total weight of said pharmaceutical composition.

The filler in the present invention also acts as a binder and a partial disintegrant. During the research process, it was found that after using a combination of mannitol and microcrystalline cellulose, the appearance and properties of the granules can be effectively improved during dry granulation, which is more conducive to the implementation of subsequent processes. Moreover, using the combination of mannitol and microcrystalline cellulose in an appropriate ratio can improve and maintain the dissolution behavior of the product, especially in stability studies, where this phenomenon is more pronounced. Preferably, the weight ratio of mannitol to microcrystalline cellulose is from 1:0.25 to 4.

The pharmaceutical composition in the present invention may comprise a disintegrant. In one embodiment, said disintegrant can be one or more selected from croscarmellose sodium, crospovidone, sodium starch glycolate, corn starch, microcrystalline cellulose and the combination thereof. Preferably, one or more of croscarmellose sodium, crospovidone, polyvinylpyrrolidone, microcrystalline cellulose and the combination thereof can be used.

In one embodiment, said disintegrant can be croscarmellose sodium. The amount is from 0.5 wt% to 20.0 wt%, based on the total weight of said pharmaceutical composition.

The pharmaceutical composition in the present invention may comprise a glidant. In one embodiment, the glidant can be selected from silicon dioxide and/or talc powders.

In one embodiment, said glidant can be silicon dioxide, used in an amount of 0.1 wt% to 10.0 wt%, based on the total weight of said pharmaceutical composition.

The pharmaceutical composition in the present invention may comprise a lubricant. In one embodiment, said lubricant can be one or more selected from magnesium stearate, magnesium lauryl stearate, sodium stearyl fumarate, stearic acid, calcium stearate, zinc stearate, potassium benzoate, sodium benzoate, myristic acid, palmitic acid, mineral oil, hydrogenated castor oil, medium-chain triglycerides, poloxamers, polyethylene glycols, talc powders, and the combination thereof. Preferably, one or more of magnesium stearate, sodium stearyl fumarate and the combination thereof can be used.

In one embodiment, said lubricant can be magnesium stearate, used in an amount of 0.05 wt% to 2.0 wt%, based on the total weight of said pharmaceutical composition.

In one embodiment, said pharmaceutical composition comprises, based on the total weight of said pharmaceutical composition, the following components:
0.1 wt% to 30.0 wt% of Compound 1;
1.0 wt% to 90.0 wt% of copovidone;
0 wt% to 90.0 wt% of mannitol;
0 wt% to 90.0 wt% of microcrystalline cellulose;
0.5 wt% to 20.0 wt% of croscarmellose sodium;
0.1 wt% to 10.0 wt% of sodium lauryl sulfate;
0.1 wt% to 10.0 wt% of silicon dioxide;
0.05 wt% to 2.0 wt% of magnesium stearate.

In one embodiment, a pharmaceutical composition comprising Compound 1 is provided, wherein said pharmaceutical composition comprises, based on the total weight of said pharmaceutical composition, the following components:
0.1 wt% to 30.0 wt% of Compound 1,
1.0 wt% to 90.0 wt% of copovidone,
0 wt% to 99.0 wt% of microcrystalline cellulose,
0.5 wt% to 20.0 wt% of croscarmellose sodium,
0.1 wt% to 10.0 wt% of sodium lauryl sulfate,
0.1 wt% to 10.0 wt% of silicon dioxide,
0.05 wt% to 2.0 wt% of magnesium stearate;
and/or
0.1 wt% to 30.0 wt% of Compound 1,
1.0 wt% to 90.0 wt% of copovidone,
0 wt% to 99.0 wt% of mannitol,
0.5 wt% to 20.0 wt% of croscarmellose sodium,
0.1 wt% to 10.0 wt% of sodium lauryl sulfate,
0.1 wt% to 10.0 wt% of silicon dioxide,
0.05 wt% to 2.0 wt% of magnesium stearate;
and/or
0.1 wt% to 30.0 wt% of Compound 1,
1.0 wt% to 90.0 wt% of copovidone,
0 wt% to 99.0 wt% of mannitol,
0 wt% to 99.0 wt% of microcrystalline cellulose,
0.5 wt% to 20.0 wt% of croscarmellose sodium,
0.1 wt% to 10.0 wt% of sodium lauryl sulfate,
0.1 wt% to 10.0 wt% of silicon dioxide,
0.05 wt% to 2.0 wt% of magnesium stearate.

Said pharmaceutical composition may optionally comprise one or more of a colorant, a fragrance, a correctant to enhance its visual appeal, alter taste and odor, increase identifiability, etc., and these changes do not directly affect the therapeutic effect of the pharmaceutical composition.

Said pharmaceutical composition may also be coated to achieve the purposes of protecting the coated components, distinguishing specifications, increasing identifiability, and altering drug dissolution behavior.

On the other hand, in order to ensure the formability of said pharmaceutical composition and the convenience of use and distribution, the tablet thereof should also have an appropriate hardness, such as a hardness of 40N to 150N.

The method for testing dissolution in the present invention is the second method of the Dissolution and Release Rate Determination Method in "Chinese Pharmacopoeia", Edition 2020, Part 4, Volume 0931, specifically as follows: paddle method, 75 rpm, 900 ml of phosphate buffer medium in pH 6.8, at 37+0.5°C, and 10 ml samples are taken at 10 min, 15 min, 30 min, 45 min, 60 min, 90 min, and 120 min after dosing, filtered through a 0.45 µm polypropylene membrane, discarding the first 5 ml of filtrate. The subsequent filtrate is used to test the content of Compound 1, and the dissolution ratio is calculated based on the theoretical content.

Based on the property of Compound 1 as a JAK inhibitor, the solid dispersion and pharmaceutical composition comprising Compound 1 in the present application are also useful for the treatment or prevention of various autoimmune diseases including atopic dermatitis, prurigo nodularis, vitiligo, scleroderma, psoriasis, membranous nephropathy, ankylosing spondylitis, peripheral T-cell lymphoma, ulcerative colitis, rheumatoid arthritis, systemic lupus erythematosus, Crohn's disease and alopecia areata, and the like.

In one embodiment, said pharmaceutical composition is an oral solid preparation, including a tablet, a granule, a powder, a dry suspension, or a capsule, particularly a tablet.

For example, the tablet can be prepared using the following process:
(1) Dry granulation: Using a three-dimensional mixer, mix the solid dispersion comprising Compound 1 with the glidant, lubricant, disintegrant, surfactant and filler uniformly, and then use a dry granulator to form granules. Specifically, for example, mix the solid dispersion comprising Compound 1 with colloidal silicon dioxide, croscarmellose sodium, sodium lauryl sulfate, mannitol and microcrystalline cellulose at a mixing speed of 15 rpm for 20 min. Use a dry granulator at a main pressure of 40-45 kg/cm² to compress the material into large ribbons, then pass the crushed ribbons through a 20-mesh screen to obtain granules.
(2) Final mixing: Mix the granules obtained from dry granulation with colloidal silicon dioxide and magnesium stearate uniformly.
(3) Tableting: Use a rotary tablet press to compress the final mixed material into tablets, wherein the tablet hardness is controlled between 40N and 150N, the tablet weight variation is controlled within ±3% of the theoretical weight, and no broken, cracked, or crushed tablets are detected.
(4) Packaging: As needed, pharmaceutical-grade high-density polyethylene bottles or aluminum-aluminum blister packs can be used for packaging.

### Example 1 Solubility Test of Compound 1

The inventors tested the solubility of Compound 1 (API) in various common media. The results are shown in Table 1.

**Table 1 Solubility of Compound 1 in various solvents (25°C)**

| **Medium** | **Solubility (mg/ml)** |
|---|---|
| pH1.0 hydrochloric acid medium | 1.2407 |
| pH3.0 citric acid buffer | 2.921 |
| pH4.5 sodium acetate buffer | 0.0458 |
| pH6.8 phosphate buffer | 0.0199 |
| Purified water | 0.0225 |
| Ethanol | S<5 |
| Acetonitrile | 23.1 <S<34.7 |
| Acetone | S>30 |
| Dichloromethane | 12.45<S<24.9 |

The results showed that the solubility of Compound 1 in media of different pH is clearly pH-dependent, with higher solubility in an acidic environment and almost insoluble in neutral, alkaline environments and water. Among the common solvents used for preparing solid dispersions by spray drying, acetone has a relatively high solubility for Compound 1.

### Example 2 Solubility Test of Polymers

To prepare a solid dispersion by means of spray drying, it is first necessary to completely dissolve the polymer and Compound 1 in a suitable solvent before subsequent operations. Therefore, the inventors also tested the solubility of common polymers in low-boiling-point solvents. The results are shown in Table 2.

**Table 2 Solubility of polymers in various solvents (25°C)**

| Acetone | Copovidone (Kollidon VA64) | Hydroxypropyl methylcellulose (HPMC E5) | Povidone (PVP K30) | Hydroxypropyl methylcellulose phthalate (HPMCP HP55) | Hydroxypropyl methylcellulose acetate succinate (HPMCAS LF) |
|---|---|---|---|---|---|
| Acetone | S | I | I | S | S |
| Ethanol | S | S | s | I | I |
| Tetrahydrofuran | PS | S | I | S | S |
| Methanol | S | S | S | S | S |
| Isopropanol | S | I | S | I | I |
| Ethyl acetate | I | I | I | I | S |
| Dichloromethane | S | S | S | I | I |

| | | | | | |
|---|---|---|---|---|---|
| S indicates solubility >5%; I indicates solubility <1%; PS indicates solubility between 1% and 5%. | | | | | |

### Example 3 Evaluation of the Solubilizing Ability of Polymers for Compound 1

According to the solubility test results of Compound 1 and polymers from Examples 1 and 2, methanol was chosen to dissolve the polymers, and acetone was chosen to dissolve Compound 1. The two solutions were then mixed, and solid dispersion samples of Compound 1 with different polymers were prepared using a micro-rotary evaporation method. The solubility of these samples in pH 6.8 phosphate buffer was tested. The results are shown in Table 3.

**Table 3 Evaluation of the solubilizing ability of polymers for Compound 1 (37°C)**

| **Solid Dispersion Composition** | **Weight Ratio** | **Solubility (mg/ml)** | |
|---|---|---|---|
| | | **5 min** | **60 min** |
| Compound 1 + Kollidon VA64 | 1:2 | 0.316 | 0.319 |
| | 1:5 | 0.336 | 0.330 |
| | 1:10 | 0.332 | 0.334 |
| Compound 1 + HPMC E5 | 1:2 | 0.193 | 0.198 |
| | 1:5 | 0.190 | 0.200 |
| | 1:10 | 0.201 | 0.200 |
| Compound 1 + PVP K30 | 1:2 | 0.335 | 0.261 |
| | 1:5 | 0.362 | 0.274 |
| | 1:10 | 0.369 | 0.277 |
| Compound 1 + HPMCP HP55 | 1:2 | 0.192 | 0.255 |
| | 1:5 | 0.193 | 0.251 |
| | 1:10 | 0.099 | 0.212 |
| Compound 1 + HPMCAS LF | 1:2 | 0.196 | 0.254 |
| | 1:5 | 0.211 | 0.294 |
| | 1:10 | 0.095 | 0.200 |

From the above results, it can be seen that all tested polymers effectively increased the solubility of Compound 1 in pH 6.8 phosphate buffer and effectively maintained the supersaturated state of Compound 1 in the medium.

Correspondingly, different types of polymers exhibit different solubilizing properties for Compound 1. The enteric materials HPMCP HP55 and HPMCAS LF showed similar solubilizing properties, i.e., increase in the ratio of the polymer will inhibit the solubility of Compound 1 under initial conditions, and the solubility of Compound 1 gradually increased with dissolution time. For PVP K30, the solubility of Compound 1 was relatively high under initial conditions, but the solubility of Compound 1 gradually decreased over time. After solubilizing Compound 1 with Kollidon VA64 and HPMC E5, the solubility remained relatively stable over different time.

Regarding the effect of the ratio of Compound 1to polymer on the solubilizing effect, it can be seen from the above results that, except for increasing the dosage of enteric coated materials HPMCP HP55 and HPMCAS LF to Weight Ratio 1:10, which resulted in a decrease in the solubility of compound 1 under initial conditions, there was no significant difference in the solubility of compound 1 among other polymers in the range of Weight Ratio 1:2-1:10.

Overall, Kollidon VA64 has a good solubilizing effect on Compound 1 and a good ability to maintain supersaturation.

### Example 4 Evaluation of the Solubilizing Ability of Surfactants for Compound 1

Under the same conditions, a small amount of water or methanol was used to dissolve the surfactant, which was then added to the mixed solution of Compound 1 and the polymer as shown in Example 3. Solid dispersion samples of Compound 1 + polymer + surfactant were similarly prepared using the micro-rotary evaporation method. The solubility of the samples in pH 6.8 phosphate buffer was tested. The results are shown in Table 4.

**Table 4 Evaluation of the effect of surfactant type on the solubilizing ability for Compound 1 (37°C)**

| **Active Pharmaceutical Ingredient** | **Polymer** | **Surfactant** | **Ratio** | **Solubility (mg/ml)** | |
|---|---|---|---|---|---|
| | | | | **5 min** | **60 min** |
| | VA64 | / | 1:3 | 0.323 | 0.331 |
| | | Sodium lauryl sulfate (SDS) | 1:3:0.3 | 1.456 | 1.477 |
| | | Vitamin E polyethylene glycol succinate | 1:3:0.3 | 0.467 | 0.509 |
| | | Poloxamer | 1:3:0.3 | 0.279 | 0.299 |
| API | | Polyethylene glycol 15-hydroxystearate | 1:3:0.3 | 0.392 | 0.392 |
| | HPMCP HP55 | / | 1:3 | 0.200 | 0.212 |
| | | SDS | 1:3:0.3 | 0.948 | 1.103 |
| | HPMCAS LF | / | 1:3 | 0.201 | 0.231 |
| | | SDS | 1:3:0.3 | 1.007 | 1.122 |

Comparing the above results, it can be seen that under the same conditions, sodium lauryl sulfate (SDS) has a clear solubilizing ability for Compound 1, increasing the solubility of Compound 1 very effectively and maintaining its supersaturated state for a longer time.

Based on the above results, the effect of the SDS ratio on the solubilizing ability for Compound 1 was further evaluated. The results are shown in Table 5.

**Table 5 Evaluation of the effect of surfactant ratio on the solubilizing ability for Compound 1 (37°C)**

| **Active Pharmaceutical Ingredient** | **Polymer** | **Surfactant** | **Ratio** | **Solubility (mg/ml)** | |
|---|---|---|---|---|---|
| | | | | **5 min** | **60min** |
| | | | 1:3:0.004 | 0.515 | 0.510 |
| API | VA64 | SDS | 1:3:0.04 | 0.896 | 0.903 |
| | | | 1:3:0.4 | 1.716 | 1.720 |

From the results in Table 5, it can be seen that adding SDS to the solid dispersion of Compound 1 can effectively increase the solubility of the compound. And in appropriate situations, the amount of SDS in the formulation should be increased.

Based on the above results, the effect of the way of SDS addition on the solubilizing ability for Compound 1 was further evaluated. The results are shown in Table 6.

**Table 6 Evaluation of the effect of the way of surfactant addition on the solubilizing ability for Compound 1 (37°C)**

| **Active Pharmaceutical Ingredient** | **Polymer** | **Surfactant** | **Ratio** | **Solubility (mg/ml)** | | **Way of Surfactant Addition** |
|---|---|---|---|---|---|---|
| | | | | **5 min** | **60min** | |
| | | | | 1.689 | 1.692 | Added during preparation of Compound 1 solid dispersion |
| API | VA64 | SDS | 1:3:0.4 | 1.547 | 1.545 | Solid dispersion of Compound 1 prepared first, then SDS added to the solid dispersion and mixed thoroughly and uniformly |

The results show that the process of mixing powder directly at the same SDS ratio is also beneficial for the solubilization of Compound 1.

### Example 5 Effect of Dosage Form on Exposure in vivo of Compound 1

Pharmacokinetic (PK) tests were conducted using three preparation s: a conventional tablet comprising micronized Compound 1 directly mixed and compressed (particle size controlled to D90 ≤ 5 µm) (Table 7), a conventional tablet comprising a solid dispersion of Compound 1 (Table 8), and an enteric-coated tablet comprising a solid dispersion of Compound 1 (Table 9).

The PK test method is: using beagle dogs aged 1-3 years, a single oral dose of 50 mg of the preparation was administered, and blood was collected at 5 min, 15 min, 30 min, 1 h, 2 h, 4 h, 8 h, and 24 h after administration to test the concentration of Compound 1 in the blood. A graph of time versus blood drug concentration was plotted to evaluate the changes of Compound 1 in beagle dogs.

**Table 7 Formulation composition of Compound 1 for PK test (conventional tablet comprising Compound 1)**

| **Formulation 1** | |
|---|---|
| Formulation composition | Ratio (%) |
| Compound 1 | 8.33 |
| Copovidone VA64 | 25 |
| Vitamin E polyethylene glycol succinate | 4.17 |
| Sodium lauryl sulfate | 4.17 |
| Lactose | 25.12 |
| Microcrystalline cellulose | 25.12 |
| Sodium starch glycolate | 5.09 |
| Colloidal silicon dioxide | 1 |
| Sodium stearyl fumarate | 1 |
| Magnesium stearate | 1 |
| Total weight | 100 |

**Table 8. Formulation composition of Compound 1 for PK test (conventional tablet comprising solid dispersion of Compound 1)**

| **Formulation 2** | |
|---|---|
| Formulation composition | Ratio (%) |
| Compound 1 | 8.33 |
| Copovidone VA64 | 25 |
| Vitamin E polyethylene glycol succinate | 4.17 |
| Sodium lauryl sulfate | 4.17 |
| Lactose | 25.12 |
| Microcrystalline cellulose | 25.12 |
| Sodium starch glycolate | 5.09 |
| Colloidal silicon dioxide | 1 |
| Sodium stearyl fumarate | 1 |
| Magnesium stearate | 1 |
| Total weight | 100 |

**Table 9 Formulation composition of Compound 1 for PK test (enteric-coated tablet comprising solid dispersion of Compound 1)**

| **Formulation 3** | |
|---|---|
| Formulation composition | Ratio (%) |
| Compound 1 | 8.33 |
| Copovidone | 12.5 |
| hydroxypropyl methylcellulose phthalate HP55 | 12.5 |
| Sodium lauryl sulfate | 4.17 |
| Lactose | 27 |
| Microcrystalline cellulose | 27 |
| Sodium starch glycolate | 5.5 |
| Colloidal silicon dioxide | 1 |
| Sodium stearyl fumarate | 1 |
| Magnesium stearate | 1 |
| Total weight | 100 |

In Formulations 2 and 3 above, acetone was chosen to dissolve copovidone and Compound 1, and the dissolved material was spray-dried under conditions of an inlet air temperature of 100°C and an outlet air temperature of 50°C to prepare a solid dispersion of Compound 1 and copovidone. Thereafter, it was mixed with other excipients and compressed into tablets. Formulation 1 was prepared by directly mixing Compound 1 with all excipients and compressing into tablets.

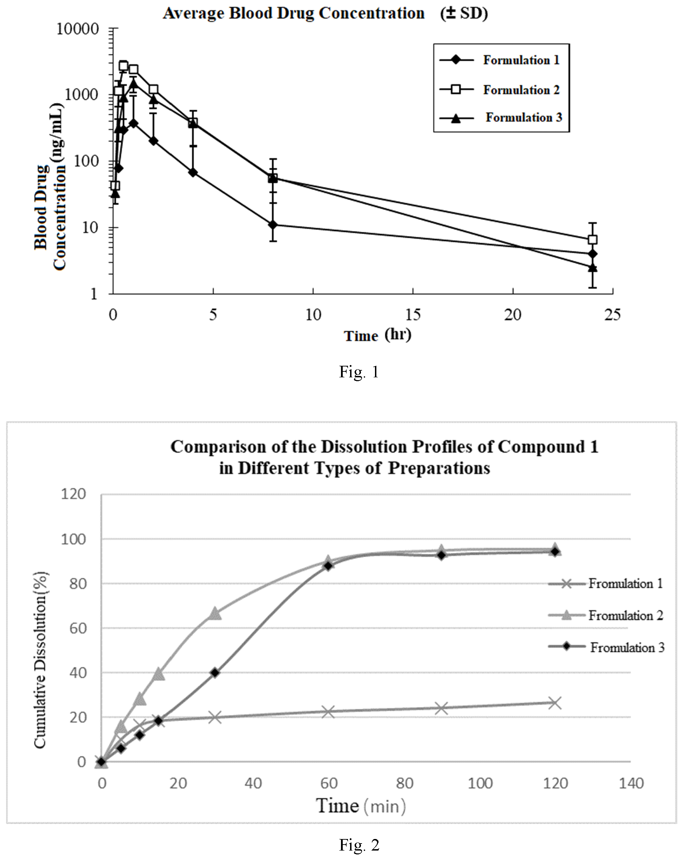

The PK results of the above pharmaceutical compositions comprising Compound 1 in beagle dogs *in vivo* are shown in Figure 1. The results showed that for Formulations 2 and 3 prepared using the solid dispersion technology, the peak concentration (Cₘₐₓ) of Compound 1 was significantly higher than that of Formulation 1 comprising micronized Compound 1 by direct physical mixing. This indicates that the solid dispersion technology effectively increases the *in vivo* exposure of Compound 1, allowing Compound 1 to more readily reach the concentration required to treat diseases. For Formulations 2 and 3, Formulation 2 exhibited a higher Cₘₐₓ and a shorter time to peak (tₘₐₓ) of the blood drug concentration, allowing the product to exert its therapeutic effect earlier after administration.

In summary, the preparation prepared using copovidone VA64 exhibited a smaller tₘₐₓ and a larger Cₘₐₓ, both of which are more favorable for the therapeutic effect of the product.

The dissolution profiles of the above three formulations were tested, and the results are shown in Figure 2. It can be seen that the dissolution of the three preparation s showed a relatively consistent trend with the PK test results in beagle dogs. In the dissolution profiles, Formulation 2 also exhibited a faster dissolution rate and reached the dissolution plateau more quickly.

### Example 6 Selection of Fillers

The effect of fillers at different ratios on product dissolution and stability was evaluated.

Preparation process:
(1) Preparation of solid dispersion sample: After Compound 1 and copovidone VA64 were dissolved in acetone, the dissolved material was spray-dried under conditions of an inlet air temperature of 100°C and an outlet air temperature of 50°C to prepare a solid dispersion.
(2) Dry granulation: The solid dispersion comprising Compound 1 was mixed with colloidal silicon dioxide, croscarmellose sodium, sodium lauryl sulfate, mannitol, and microcrystalline cellulose at a mixing speed of 15 rpm for 20 min. A dry granulator was used at a main pressure of 40-45 kg/cm² to compress the material into large ribbons, then pass the crushed ribbons through a 20-mesh screen to obtain granules.
(3) Final mixing: The granules obtained from dry granulation were mixed uniformly with colloidal silicon dioxide and magnesium stearate.
(4) Tabletting: A rotary tablet press was used to compress the final mixed material into tablets, wherein the tablet hardness was controlled between 40N and 150N, the tablet weight variation was controlled within ±3% of the theoretical weight, and no broken, cracked, or crushed tablets were detected.

The formulation design for filler selection is shown in Table 10.

**Table 10 Formulation design for filler selection**

| **Formulation No.** | | **For.* 4** | **For. 5** | **For. 6** | **For. 7** | **For. 8** | **For. 9** | **For. 10** | **For. 11** | **For. 12** |
|---|---|---|---|---|---|---|---|---|---|---|
| Formulation Composition | | Ratio (%) | | | | | | | | |
| Solid disper sion | Compound 1 | 1 | 10 | 20 | 1 | 10 | 20 | 10 | 10 | 10 |
| | Copovidone VA64 | 3 | 30 | 60 | 3 | 30 | 60 | 30 | 30 | 30 |
| Mannitol | | 0 | 0 | 0 | 86 | 50 | 10 | 5 | 25 | 45 |
| Microcrystalline cellulose | | 86 | 50 | 10 | 0 | 0 | 0 | 45 | 25 | 5 |
| Croscarmellose sodium | | 5 | 5 | 5 | 5 | 5 | 5 | 5 | 5 | 5 |
| Sodium lauryl sulfate | | 2 | 2 | 2 | 2 | 2 | 2 | 2 | 2 | 2 |
| Colloidal silicon dioxide (intragranular) | | 1 | 1 | 1 | 1 | 1 | 1 | 1 | 1 | 1 |
| Colloidal silicon dioxide (extragranular) | | 1 | 1 | 1 | 1 | 1 | 1 | 1 | 1 | 1 |
| Magnesium stearate (intragranular) | | 0.5 | 0.5 | 0.5 | 0.5 | 0.5 | 0.5 | 0.5 | 0.5 | 0.5 |
| Magnesium stearate (extragranular) | | 0.5 | 0.5 | 0.5 | 0.5 | 0.5 | 0.5 | 0.5 | 0.5 | 0.5 |
| Total | | 100 | 100 | 100 | 100 | 100 | 100 | 100 | 100 | 100 |

| | | | | | | | | | | |
|---|---|---|---|---|---|---|---|---|---|---|
| *: " For." is the abbreviated form of "Formulation". The same applies hereinafter. "Intragranular" means added in step (2) dry granulation; "extragranular" means added in step (3) final mixing. Similarly hereinafter. | | | | | | | | | | |

The stability test results of the above formulations under accelerated conditions are shown in Table 11.

**Table 11 Effect of fillers on the stability of Compound 1 preparation s**

| **Formulation** | **Compound 1** | **For. 4** | **For. 5** | **For. 6** | **For. 7** | **For. 8** | **For. 9** | **For. 10** | **For. 11** | **For. 12** |
|---|---|---|---|---|---|---|---|---|---|---|
| Condition | | 40°C, RH75%, 1 month | | | | | | | | |
| Largest single impurity (%) | 0.11 | 0.11 | 0.11 | 0.10 | 0.11 | 0.11 | 0.11 | 0.11 | 0.11 | 0.11 |
| Total impurities (%) | 0.42 | 0.42 | 0.42 | 0.41 | 0.41 | 0.41 | 0.42 | 0.42 | 0.42 | 0.42 |

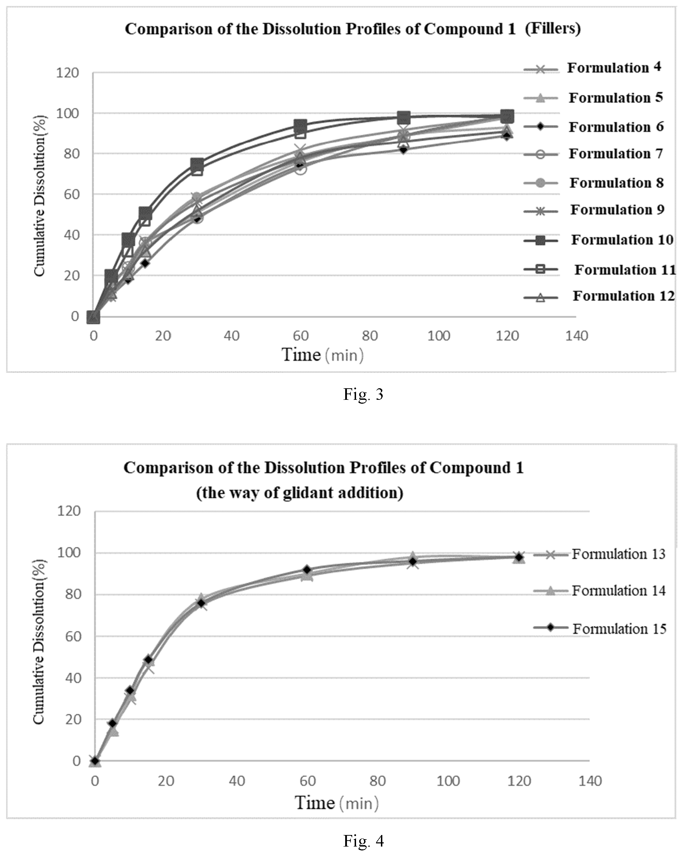

By comparing the above results, it can be seen that the total amount of mannitol and microcrystalline cellulose ranges from 0% to 86%, and all preparation s achieved more than 80% dissolution within 90 minutes and complete dissolution within 120 minutes, as shown in Figure 3. Correspondingly, when mannitol and microcrystalline cellulose were used at the same time and maintained in similar amounts, the dissolution of the preparation showed a faster trend.

### Example 7 Selection of Glidants

The effect of the amount and the way of addition of silicon dioxide on product preparation process and dissolution was evaluated.

First, the effect of silicon dioxide on the dry granulation process was evaluated. The process feasibility was evaluated by adding or not adding silicon dioxide during the dry granulation process (as shown in Table 12). The process was the same as the preparation process in Example 6. The results are shown in Table 13.

**Table 12 Formulation design for evaluating the effect of the way of glidant addition**

| **Formulation No.** | | **Formulation 13** | **Formulation 14** | **Formulation 15** |
|---|---|---|---|---|
| Formulation Composition | | Ratio (%) | Ratio (%) | Ratio (%) |
| Solid dispersion | Compound 1 | 10 | 10 | 10 |
| | Copovidone VA64 | 30 | 30 | 30 |
| Mannitol | | 25 | 25 | 25 |
| Microcrystalline cellulose | | 25 | 25 | 25 |
| Croscarmellose sodium | | 5 | 5 | 5 |
| Sodium lauryl sulfate | | 2 | 2 | 2 |
| Colloidal silicon dioxide (intragranular) | | 0 | 1 | 2 |
| Colloidal silicon dioxide (extragranular) | | 2 | 1 | 0 |
| Magnesium stearate (intragranular) | | 0.5 | 0.5 | 0.5 |
| Magnesium stearate (extragranular) | | 0.5 | 0.5 | 0.5 |
| Total | | 100 | 100 | 100 |

**Table 13 Effect of the way of glidant addition on dry granulation (dry granulation)**

| **Process Stage** | **Evaluation Item** | **Formulation 13** | **Formulation 14** | **Formulation 15** |
|---|---|---|---|---|
| | Material flowability | Moderate | Good | Good |
| Dry granulation | Susceptibility to sticking on rollers or not | Occasional | No | No |
| | Risk of continuous production | At risk | Low risk | Low risk |

From the above results, it can be seen that during the dry granulation process, when removing the intragranular silicon dioxide, due to decreased material flowability, sticking on rollers easily occurred, leading to reduced feasibility of continuous production. Therefore, in order to ensure ease of industrial production, the role of intragranular glidant is clear.

The above granules were used in the tabletting process to evaluate the effect of extragranular glidant. The results are shown in Table 14.

**Table 14 Effect of the way of glidant addition on dry granulation (tabletting)**

| **Process Stage** | **Evaluation Item** | **Formulation 13** | **Formulation 14** | **Formulation 15** |
|---|---|---|---|---|
| Tabletting process | Material flowability | Good | Good | Moderate |
| | Risk of continuous production | Low risk | Low risk | At risk |

From the above results, it can be seen that extragranular silicon dioxide also helps increase the flowability of the granules, ensuring smooth continuous production.

The effect of the way of glidant addition on preparation dissolution is shown in Figure 4. It can be seen that different ways of glidant addition have no significant effect on preparation dissolution.

The effect of the amount of glidants on the preparation process and dissolution was further evaluated, as shown in Tables 15 and 16.

**Table 15 Formulation design for evaluating the effect of the amount of glidants**

| **Formulation No.** | | **Formulation 16** | **Formulation 17** | **Formulation 18** |
|---|---|---|---|---|
| Formulation Composition | | Ratio (%) | Ratio (%) | Ratio (%) |
| Solid dispersion | Compound 1 | 10 | 10 | 10 |
| | Copovidone VA64 | 30 | 30 | 30 |
| Mannitol | | 25 | 25 | 25 |
| Microcrystalline cellulose | | 26.7 | 22 | 17 |
| Croscarmellose sodium | | 5 | 5 | 5 |
| Sodium lauryl sulfate | | 2 | 2 | 2 |
| Colloidal silicon dioxide (intragranular) | | 0.15 | 2.5 | 5 |
| Colloidal silicon dioxide (extragranular) | | 0.15 | 2.5 | 5 |
| Magnesium stearate (intragranular) | | 0.5 | 0.5 | 0.5 |
| Magnesium stearate (extragranular) | | 0.5 | 0.5 | 0.5 |
| Total | | 100 | 100 | 100 |

**Table 16 Effect of the amount of glidants on preparation process**

| **Process Stage** | **Evaluation Item** | **Formulation 16** | **Formulation 17** | **Formulation 18** |
|---|---|---|---|---|
| Dry granulation | Material flowability | Good | Good | Good |
| | Susceptibility to sticking on rollers or not | No | No | No |
| | Risk of continuous production | Low risk | Low risk | Low risk |
| Tabletting process | Material flowability | Good | Good | Good |
| | Risk of continuous production | Low risk | Low risk | Low risk |

From the above results, it can be seen that the amount of glidants in the range of 0.3%-10% does not significantly affect the preparation process.

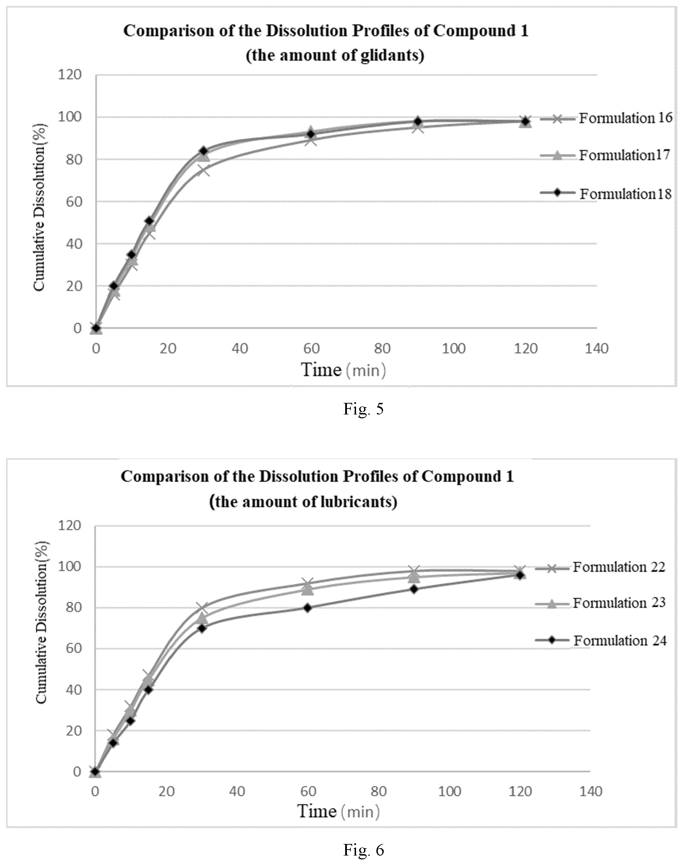

The effect of the amount of glidants on preparation dissolution is shown in Figure 5. It can be seen that within the range of 0.3%-10%, the amount of glidants does not significantly affect the dissolution behavior of the preparation.

### Example 8 Selection of Lubricants

The effect of the amount and addition way of lubricants on the process and dissolution of the product preparation was evaluated.

First, the effect of the lubricant on the dry granulation process was evaluated: the feasibility of the process was evaluated by adding magnesium stearate or not during the dry granulation process. The process was the same as the preparation process in Example 6, as shown in Tables 17 and 18.

**Table 17 Formulation design for evaluating the effect of the way of lubricant addition**

| **Formulation No.** | | **Formulation 19** | **Formulation 20** | **Formulation 21** |
|---|---|---|---|---|
| Formulation Composition | | Ratio (%) | Ratio (%) | Ratio (%) |
| Solid dispersion | Compound 1 | 10 | 10 | 10 |
| | Copovidone VA64 | 30 | 30 | 30 |
| Mannitol | | 25 | 25 | 25 |
| Microcrystalline cellulose | | 25 | 25 | 25 |
| Croscarmellose sodium | | 5 | 5 | 5 |
| Sodium lauryl sulfate | | 2 | 2 | 2 |
| Colloidal silicon dioxide (intragranular) | | 1 | 1 | 1 |
| Colloidal silicon dioxide (extragranular) | | 1 | 1 | 1 |
| Magnesium stearate (intragranular) | | 0 | 0.5 | 1 |
| Magnesium stearate (extragranular) | | 1 | 0.5 | 0 |
| Total | | 100 | 100 | 100 |

**Table 18 Effect of the amount of lubricants on preparation process**

| **Process Stage** | **Evaluation Item** | **Formulation 19** | **Formulation 20** | **Formulation 21** |
|---|---|---|---|---|
| Dry granulation | Material flowability | Moderate | Good | Good |
| | Susceptibility to sticking on rollers or not | Yes | No | No |
| | Risk of continuous production | At risk | Low risk | Low risk |
| Tabletting process | Risk of sticking to punches | Not observed | Not observed | Yes |
| | Risk of continuous production | Low risk | Low risk | At risk |

During the preparation production process, it was found that adding the lubricant either in the dry granulation or in the tabletting process did not result in good preparation process feasibility, but after adding the lubricant in both processes, the process feasibility of the sample was good.

The formulation design in Table 19 was used to further evaluate the effect of the amount of lubricants on preparation dissolution.

**Table 19 Formulation design for evaluating the effect of the amount of lubricants**

| **Formulation No.** | | **Formulation 22** | **Formulation 23** | **Formulation 24** |
|---|---|---|---|---|
| Formulation Composition | | Ratio (%) | Ratio (%) | Ratio (%) |
| Solid dispersion | Compound 1 | 10 | 10 | 10 |
| | Copovidone VA64 | 30 | 30 | 30 |
| Mannitol | | 25 | 25 | 25 |
| Microcrystalline cellulose | | 25.9 | 25 | 24 |
| Croscarmellose sodium | | 5 | 5 | 5 |
| Sodium lauryl sulfate | | 2 | 2 | 2 |
| Colloidal silicon dioxide (intragranular) | | 1 | 1 | 1 |
| Colloidal silicon dioxide (extragranular) | | 1 | 1 | 1 |
| Magnesium stearate (intragranular) | | 0.05 | 0.5 | 1 |
| Magnesium stearate (extragranular) | | 0.05 | 0.5 | 1 |
| Total | | 100 | 100 | 100 |

The results of the effect of lubricant amounts on preparation dissolution are shown in Figure 6. It can be seen that within the range of 0.05%-2%, as the amount of lubricants increases, the dissolution rate of the preparation tends to decrease. Therefore, the amount of lubricants should be controlled not to exceed the above range to avoid the effect of over-lubrication on preparation quality.

### Example 9 Selection of Disintegrants

The formulation design in Table 20 was used to evaluate the effect of different amounts of disintegrants on the dissolution of Compound.

The preparation process was the same as that shown in Example 6.

**Table 20 Formulation design for disintegrant selection**

| **Formulation No.** | | **For. 25** | **For. 26** | **For. 27** | **For. 28** | **For. 29** |
|---|---|---|---|---|---|---|
| Formulation Composition | | Ratio (%) | Ratio (%) | Ratio (%) | Ratio (%) | Ratio (%) |
| Solid dispersion | Compound 1 | 10 | 10 | 10 | 10 | 10 |
| | Copovidone VA64 | 30 | 30 | 30 | 30 | 30 |
| Mannitol | | 25 | 25 | 25 | 25 | 25 |
| Microcrystalline cellulose | | 29.5 | 25 | 20 | 15 | 10 |
| Croscarmellose sodium | | 0.5 | 5 | 10 | 15 | 20 |
| Sodium lauryl sulfate | | 2 | 2 | 2 | 2 | 2 |
| Colloidal silicon dioxide (intragranular) | | 1 | 1 | 1 | 1 | 1 |
| Colloidal silicon dioxide (extragranular) | | 1 | 1 | 1 | 1 | 1 |
| Magnesium stearate (intragranular) | | 0.5 | 0.5 | 0.5 | 0.5 | 0.5 |
| Magnesium stearate (extragranular) | | 0.5 | 0.5 | 0.5 | 0.5 | 0.5 |
| Total | | 100 | 100 | 100 | 100 | 100 |

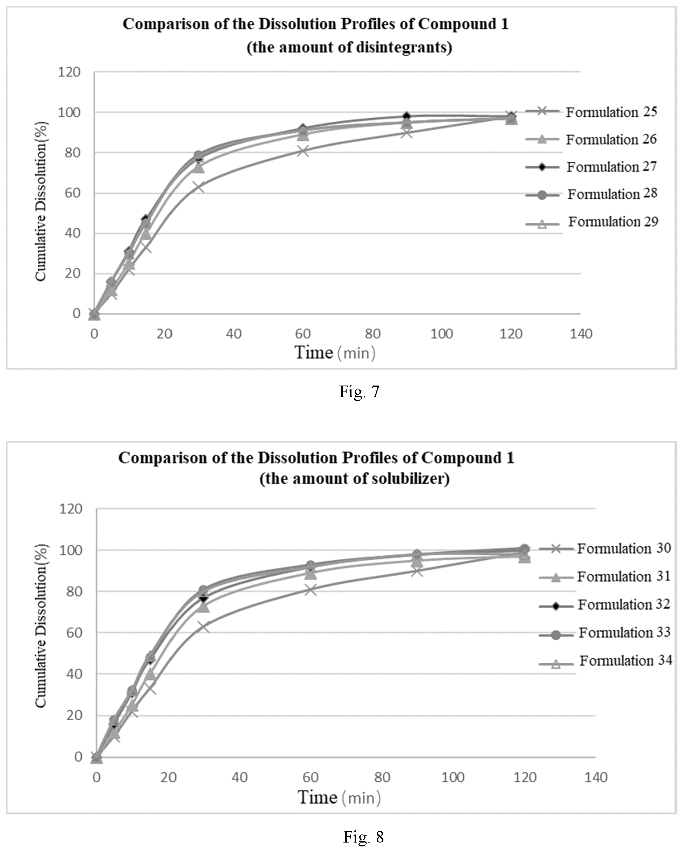

The results of the effect of different disintegrant amounts on the dissolution profile of Compound 1 are shown in Figure 7. It can be seen that when the amount of disintegrants is small (≤5%), the dissolution rate of the preparation is relatively slow. When the amount of disintegrants is large (≥15%), the dissolution rate of the preparation does not continue to increase with the increase of disintegrants.

### Example 10 Study on Surfactant in the Complete Formulation

Using the formulation design in Table 21, the effect of sodium lauryl sulfate (SDS) on product dissolution was evaluated in the complete formulation. The preparation process was the same as that shown in Example 6.

**Table 21 Formulation design for surfactant evaluation**

| **Formulation No.** | | **For. 30** | **For. 31** | **For. 32** | **For. 33** | **For. 34** |
|---|---|---|---|---|---|---|
| Formulation Composition | | Ratio (%) | Ratio (%) | Ratio (%) | Ratio (%) | Ratio (%) |
| Solid dispersion | Compound 1 | 10 | 10 | 10 | 10 | 10 |
| | Copovidone VA64 | 30 | 30 | 30 | 30 | 30 |
| Mannitol | | 20 | 20 | 20 | 20 | 20 |
| Microcrystalline cellulose | | 26.8 | 26 | 25 | 22 | 17 |
| Croscarmellose sodium | | 10 | 10 | 10 | 10 | 10 |
| Sodium lauryl sulfate* | | 0.2 | 1 | 2 | 5 | 10 |
| Colloidal silicon dioxide (intragranular) | | 1 | 1 | 1 | 1 | 1 |
| Colloidal silicon dioxide (extragranular) | | 1 | 1 | 1 | 1 | 1 |
| Magnesium stearate (intragranular) | | 0.5 | 0.5 | 0.5 | 0.5 | 0.5 |
| Magnesium stearate (extragranular) | | 0.5 | 0.5 | 0.5 | 0.5 | 0.5 |
| Total | | 100 | 100 | 100 | 100 | 100 |

| | | | | | | |
|---|---|---|---|---|---|---|
| *The solid dispersion does not contain the surfactant SDS. The solid dispersion is combined with the surfactant SDS and other excipients to prepare tablets. | | | | | | |

The results of the effect of the surfactant SDS on the dissolution of Compound 1 are shown in Figure 8. It can be seen that the dissolution rate of Compound 1 gradually increases with the increase of the amount of SDS until the amount of SDS reaches 5%, after which the increase in dissolution rate slows down. Therefore, while ensuring clinical safety, the amount of SDS should be appropriately increased to ensure rapid dissolution of the product.

### Example 11 Selection of the Hardness of Preparation

Using the formulation design in Table 22, the hardness of tablets, the key preparation quality attribute, was also evaluated during preparation and process studies.

**Table 22 Formulation design for evaluation the hardness of preparations**

| **Formulation No.** | | **Formulation 35** | **Formulation 36** | **Formulation 37** | **Formulation 38** |
|---|---|---|---|---|---|
| Formulation Composition | | Ratio (%) | Ratio (%) | Ratio (%) | Ratio (%) |
| Solid dispersion | Compound 1 | 10 | 10 | 10 | 10 |
| | Copovidone VA64 | 30 | 30 | 30 | 30 |
| Mannitol | | 20 | 20 | 20 | 20 |
| Microcrystalline cellulose | | 22 | 22 | 22 | 22 |
| Croscarmellose sodium | | 10 | 10 | 10 | 10 |
| Sodium lauryl sulfate | | 5 | 5 | 5 | 5 |
| Colloidal silicon dioxide (intragranular) | | 1 | 1 | 1 | 1 |
| Colloidal silicon dioxide (extragranular) | | 1 | 1 | 1 | 1 |
| Magnesium stearate (intragranular) | | 0.5 | 0.5 | 0.5 | 0.5 |
| Magnesium stearate (extragranular) | | 0.5 | 0.5 | 0.5 | 0.5 |
| Total | | 100 | 100 | 100 | 100 |
| Average hardness of Preparations (N) | | 46 | 79 | 95 | 139 |

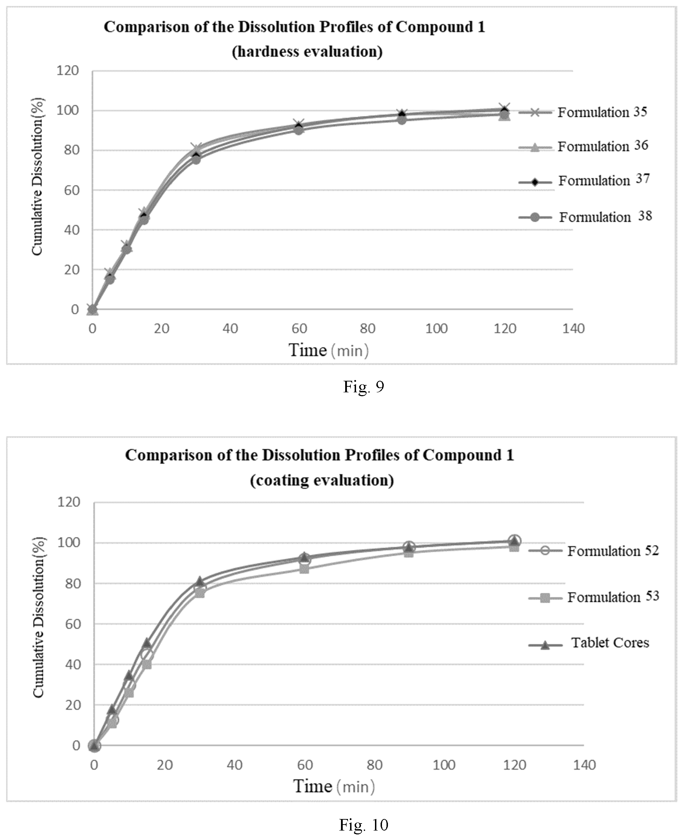

The effect of product hardness on the dissolution of Compound 1 is shown in Figure 9. It can be seen that within the tested range of 40-150N, the dissolution of the preparation is less affected by hardness.

### Example 12 Study on Specifications of Preparations

The formulation composition of the present invention mainly comprises two parts: one part is the solid dispersion comprising Compound 1, and the other part is excipients without the active ingredient. Therefore, when determining the dosage form and specifications of the product, the solid dispersion can be directly prepared into an individual product using an appropriate process, such as a powder, a granule, a capsule, a tablet, etc. Alternatively, other excipients can be added to the solid dispersion before preparing the corresponding dosage form. Therefore, the specifications of the product can be flexibly set. Tables 23 and 24 below list the formulation compositions of the product in several specifications. The preparation process was the same as that in Example 6. Since the composition of all samples was similar, some samples were selected for stability testing. The results are shown in Table 25.

**Table 23 Examples of selection of preparation specifications (1)**

| **Formulation No.** | | **For. 39** | **For. 40** | **For. 41** | **For. 42** | **For. 43** | **For. 44** | **For. 45** |
|---|---|---|---|---|---|---|---|---|
| Formulation Composition | | Weight (mg) | Weight (mg) | Weight (mg) | Weight (mg) | Weight (mg) | Weight (mg) | Weight (mg) |
| Solid dispersion | Compound 1 | 5 | 15 | 45 | 75 | 100 | 150 | 250 |
| | Copovidone VA64 | 15 | 45 | 135 | 225 | 300 | 450 | 750 |
| Mannitol | | 10 | 30 | 90 | 150 | 90 | 90 | 90 |
| Microcrystalline cellulose | | 11 | 33 | 99 | 165 | 99 | 99 | 99 |
| Croscarmellose sodium | | 5 | 15 | 45 | 75 | 45 | 45 | 45 |
| Sodium lauryl sulfate | | 2.5 | 7.5 | 22.5 | 37.5 | 22.5 | 22.5 | 22.5 |
| Colloidal silicon dioxide (intragranular) | | 0.5 | 1.5 | 4.5 | 7.5 | 4.5 | 4.5 | 4.5 |
| Colloidal silicon dioxide (extragranular) | | 0.5 | 1.5 | 4.5 | 7.5 | 4.5 | 4.5 | 4.5 |
| Magnesium stearate (intragranular) | | 0.25 | 0.75 | 2.25 | 3.75 | 2.25 | 2.25 | 2.25 |
| Magnesium stearate (extragranular) | | 0.25 | 0.75 | 2.25 | 3.75 | 2.25 | 2.25 | 2.25 |

**Table 24 Examples of selection of preparation specifications (2)**

| **Formulation No.** | | **For. 46** | **For. 47** | **For. 48** | **For. 49** | **For. 50** | **For. 51** |
|---|---|---|---|---|---|---|---|
| Formulation Composition | | Weight (mg) | Weight (mg) | Weight (mg) | Weight (mg) | Weight (mg) | Weight (mg) |
| Solid dispersion | Compound 1 | 0.5 | 0.75 | 1 | 2 | 3 | 4 |
| | Copovidone VA64 | 5 | 7.5 | 10 | 10 | 15 | 20 |
| Mannitol | | 37 | 37 | 37 | 37 | 55.5 | 74 |
| Microcrystalline cellulose | | 37 | 37 | 37 | 37 | 55.5 | 74 |
| Croscarmellose sodium | | 5 | 5 | 5 | 5 | 7.5 | 10 |
| Sodium lauryl sulfate | | 5 | 5 | 5 | 5 | 7.5 | 10 |
| Colloidal silicon dioxide (intragranular) | | 1 | 1 | 1 | 1 | 1.5 | 2 |
| Colloidal silicon dioxide (extragranular) | | 1 | 1 | 1 | 1 | 1.5 | 2 |
| Magnesium stearate (intragranular) | | 1 | 1 | 1 | 1 | 1.5 | 2 |
| Magnesium stearate (extragranular) | | 1 | 1 | 1 | 1 | 1.5 | 2 |

**Table 25 Stability test results of preparations**

| **Formulation** | **Compound 1** | **For. 39** | **For. 43** | **For. 45** | **For. 46** | **For. 49** | **For. 51** |
|---|---|---|---|---|---|---|---|
| Condition | | 40°C, RH75%, 1 month | | | | | |
| Largest single impurity (%) | 0.10 | 0.10 | 0.11 | 0.10 | 0.10 | 0.10 | 0.10 |
| Total impurities (%) | 0.38 | 0.38 | 0.39 | 0.38 | 0.38 | 0.38 | 0.38 |

From the above results, it can be seen that there is no significant difference in the stability of products with different specifications. The preparation specifications can be reasonably selected according to clinical treatment needs.

### Example 13 Study on the Effect of Coating on Preparations

The product of the present invention can be coated as needed. The coating material can be a conventional non-functional film coating material to increase identifiability, distinguish product specifications, etc.; or it can be a functional coating material, such as using a functional coating material to give the product characteristics of site-specific, time-specific, or rate-specific release. Coating can also increase the stability of the tablet core, such as reducing the impact of light, moisture, etc., on the tablet core.

Using the formulation design in Table 26, the effect of coating on product dissolution and stability was tested using a film coating and an enteric coating, respectively.

The preparation process for the tablet cores was the same as that shown in Example 6.

The coating process was as follows: prepare a coating solution with a solid content of 10% by dissolving the coating powder in purified water, and coat the tablet cores until the average coating weight gain reached the theoretical amount. After coating is completed, the product moisture content should be controlled to no more than 5%.

**Table 26 Formulation design for the effect of coating on preparations**

| **Composition** | **Formulation No.** | | **For. 52** | **For. 53** |
|---|---|---|---|---|
| | Formulation Composition | | Ratio (%) | Ratio (%) |
| Tablet cores | Solid dispersion | Compound 1 | 10 | 10 |
| | | Copovidone VA64 | 30 | 30 |
| | Mannitol | | 20 | 20 |
| | Microcrystalline cellulose | | 22 | 22 |
| | Croscarmellose sodium | | 10 | 10 |
| | Sodium lauryl sulfate | | 5 | 5 |
| | Colloidal silicon dioxide (intragranular) | | 1 | 1 |
| | Colloidal silicon dioxide (extragranular) | | 1 | 1 |
| | Magnesium stearate (intragranular) | | 0.5 | 0.5 |
| | Magnesium stearate (extragranular) | | 0.5 | 0.5 |
| | Total | | 100 | 100 |
| Film coating | Opadry^{®} Y-1-7000 (wt% based on tablet core) | | 2 | / |
| Enteric coating | Opadry^{®} Y-1-7000 (wt% based on tablet core) | | / | 1.5 |
| | Sureteric^{®} (wt% based on tablet core) | | / | 9 |

The stability results are shown in Table 27.

**Table 27 Effect of coating on preparation stability**

| **Formulation** | **Compound 1** | **Formulation 52** | **Formulation 53** |
|---|---|---|---|
| Condition | | 40°C, RH75%, 1 month | |
| Largest single impurity (%) | 0.10 | 0.11 | 0.10 |
| Total impurities (%) | 0.38 | 0.38 | 0.39 |

The effect of coating on product dissolution is shown in Figure 10.

From the above results, it can be seen that coating did not significantly affect the stability or dissolution of the product.

### Example 14: Effect of Solid Dispersion Preparation Process on the Product

Solid dispersions comprising Compound 1 were prepared using spray drying and hot-melt extrusion processes, respectively, and the differences between the solid dispersions obtained by the two processes were tested.

The spray drying process was the same as the preparation process shown in Example 6.

The hot-melt extrusion process was as follows:
Compound 1 and copovidone VA64 were mixed uniformly in a weight ratio of 1:9. The material was melt-extruded at 180°C by a double-screw hot-melt extruder (e.g., Pharma 11 double-screw extruder), then cooled and milled to below 40 mesh. The milled material was then mixed uniformly with the remaining excipients, and granules were prepared according to the dry granulation process shown in Example 6, followed by the subsequent steps to prepare tablets.

The formulations of the products prepared by the two processes are shown in Table 28 below.

**Table 28 Formulation composition of samples for process evaluation**

| **Formulation No.** | | **Formulation 54** | **Formulation 55** |
|---|---|---|---|
| **Process** | | **Spray drying** | **Hot-melt extrusion** |
| Formulation Composition | | Ratio (%) | Ratio (%) |
| Solid dispersion | Compound 1 | 10 | 4 |
| | Copovidone VA64 | 30 | 36 |
| Mannitol | | 20 | 20 |
| Microcrystalline cellulose | | 22 | 22 |
| Croscarmellose sodium | | 10 | 10 |
| Sodium lauryl sulfate | | 5 | 5 |
| Colloidal silicon dioxide (intragranular) | | 1 | 1 |
| Colloidal silicon dioxide (extragranular) | | 1 | 1 |
| Magnesium stearate (intragranular) | | 0.5 | 0.5 |
| Magnesium stearate (extragranular) | | 0.5 | 0.5 |
| Total | | 100 | 100 |

**Table 29 Effect of process on the solubility of Compound 1 in the solid dispersion**

| Active Pharmaceutical Ingredient | Polymer | Ratio | Solubility (mg/ml) | | Preparation Process |
|---|---|---|---|---|---|
| | | | 5 min | 60 min | |
| API | VA64 | 1:3 | 1.702 | 1.710 | Spray drying |
| | | 1:9 | 1.515 | 1.721 | Hot-melt extrusion |

**Table 30 Effect of process on preparation stability**

| Formulation No. | | Formulation 54 | | Formulation 55 | |
|---|---|---|---|---|---|
| Process | | Spray drying | | Hot-melt extrusion | |
| Condition | Compound 1 | 40°C, RH75% | | 40°C, RH75% | |
| | | 1 month | 3 months | 1 month | 3 months |
| Largest single impurity (%) | 0.11 | 0.11 | 0.13 | 0.12 | 0.13 |
| Total impurities (%) | 0.38 | 0.38 | 0.41 | 0.40 | 0.43 |

The results of solubility and stability are shown in Tables 29 and 30. From the above results, it can be seen that both of hot-melt extrusion and spray drying processes are suitable for the preparation of the solid dispersion comprising Compound 1.

### Example 15 Study on Composition of Products in Different Dosage Forms

As mentioned above, multiple intermediate products are generated during the process of making Compound 1 into tablets. These intermediate products can be further processed into a new dosage form using appropriate processes to meet the needs of different patient populations and different indications. The composition of several products was studied below.

Using the formulation compositions in Table 31, a solid dispersion comprising Compound 1 was prepared by the spray drying process shown in Example 6. The excipients below were added to the solid dispersion, mixed uniformly, and filled into double-layer aluminum-plastic composite film bags (to reduce the impact of humidity on the product) to obtain a powder suitable for patient use.

**Table 31 Powder formulation composition**

| **Dosage form** | | **Powder** |
|---|---|---|
| Formulation Composition | | Ratio (%) |
| Solid dispersion | Compound 1 | 20 |
| | Copovidone VA64 | 60 |
| Croscarmellose sodium | | 9.99 |
| Sodium lauryl sulfate | | 5 |
| Sucrose | | 5 |
| Strawberry flavor | | 0.01 |
| Total | | 100 |
| Product packaging | | Double-layer aluminum-plastic composite film bag |

Using the formulation composition in Table 32, a solid dispersion comprising Compound 1 was prepared using the spray drying process shown in Example 6. The following excipients comprising a suspending agent were added to the solid dispersion, mixed uniformly, and filled into double-layer aluminum-plastic composite film bags (to reduce the impact of humidity on the product) to obtain a dry suspension suitable for patient use.

**Table 32 Dry suspension formulation composition**

| **Dosage form** | | **Dry suspension** |
|---|---|---|
| Formulation Composition | | Ratio (%) |
| Solid dispersion | Compound 1 | 20 |
| | Copovidone VA64 | 60 |
| Croscarmellose sodium | | 9.69 |
| Sodium lauryl sulfate | | 5 |
| Xanthan gum | | 0.3 |
| Sucrose | | 5 |
| Strawberry flavor | | 0.01 |
| Total | | 100 |
| Product packaging | | Double-layer aluminum-plastic composite film bag |

Using the formulation composition in Table 33, a solid dispersion comprising Compound 1 was prepared using the spray drying process shown in Example 6. The solid dispersion was mixed uniformly with the following excipients, and granules were prepared using the same dry granulation process as shown in Example 6. The granules were filled into double-layer aluminum-plastic composite film bags (to reduce the impact of humidity on the product) to obtain granules suitable for patient use.

**Table 33 Granule formulation composition**

| **Dosage form** | | **Granule** |
|---|---|---|
| Formulation Composition | | Ratio (%) |
| Solid dispersion | Compound 1 | 10 |
| | Copovidone VA64 | 30 |
| Mannitol | | 18.49 |
| Microcrystalline cellulose | | 20 |
| Croscarmellose sodium | | 10 |
| Sodium lauryl sulfate | | 5 |
| Colloidal silicon dioxide (intragranular) | | 1 |
| Magnesium stearate (intragranular) | | 0.5 |
| Sucrose | | 5 |
| Strawberry flavor | | 0.01 |
| Total | | 100 |
| Product packaging | | Double-layer aluminum-plastic composite film bag |

Using the formulation composition in Table 34, a solid dispersion comprising Compound 1 was prepared using the spray drying process shown in Example 6. The solid dispersion was mixed uniformly with the following excipients, and granules were prepared using the same dry granulation process as shown in Example 6. The granules were filled into capsule shells to obtain capsules. The capsules can also be packaged in double-aluminum blister packs (to reduce the impact of humidity on the product) for patient convenience and storage.

**Table 34 Capsule formulation composition**

| **Dosage form** | | **Capsule** |
|---|---|---|
| Formulation Composition | | Ratio (%) |
| Solid dispersion | Compound 1 | 10 |
| | Copovidone VA64 | 30 |
| Mannitol | | 20.5 |
| Microcrystalline cellulose | | 22 |
| Croscarmellose sodium | | 10 |
| Sodium lauryl sulfate | | 5 |
| Colloidal silicon dioxide (intragranular) | | 1 |
| Magnesium stearate (intragranular) | | 0.5 |
| Colloidal silicon dioxide (extragranular) | | 1 |
| Total | | 100 |
| Capsule shell | | HPMC capsule shell |
| Outer packaging material | | Double-aluminum blister pack |

Those skilled in the art can also continue to research and develop Compound 1 preparation based on the methods described in the present invention, and such research and development will be carried out according to existing knowledge in the field and will therefore also fall within the protection scope of the present application.

### Example 16 Study on Preparation Stability

Using the formulation composition in Table 35, the preparation process was the same as in Example 6. The stability of the preparation of the present invention was tested under accelerated conditions. The test items included dissolution of preparations, related substances, crystal form, etc. The results are shown in Table 36.

**Table 35 Formulation composition of samples for stability test**

| **Formulation No.** | | **Formulation 56** |
|---|---|---|
| Formulation Composition | | Ratio (%) |
| Solid dispersion | Compound 1 | 10 |
| | Copovidone VA64 | 30 |
| Mannitol | | 20 |
| Microcrystalline cellulose | | 22 |
| Croscarmellose sodium | | 10 |
| Sodium lauryl sulfate | | 5 |
| Colloidal silicon dioxide (intragranular) | | 1 |
| Colloidal silicon dioxide (extragranular) | | 1 |
| Magnesium stearate (intragranular) | | 0.5 |
| Magnesium stearate (extragranular) | | 0.5 |
| Total | | 100 |

**Table 36 Stability test results of samples**

| **Formulation** | **Formulation 56** | | |
|---|---|---|---|
| Condition | 0 day | 40°C, RH75% | |
| | | 3 months | 6 months |
| Appearance | Off-white tablet | Off-white tablet | Off-white tablet |
| Largest single impurity (%) | 0.11 | 0.11 | 0.13 |
| Total impurities (%) | 0.38 | 0.40 | 0.43 |
| Moisture (%) | 1.68 | 1.68 | 1.69 |
| Content (%) | 99.7 | 99.7 | 99.7 |
| Total aerobic microbial count | < 10² cfu/g | / | < 10² cfu/g |
| Total combined veasts/molds count | < 10¹ cfu/g | / | < 10¹ cfu/g |
| *Escherichia coli* | Not detected | / | Not detected |

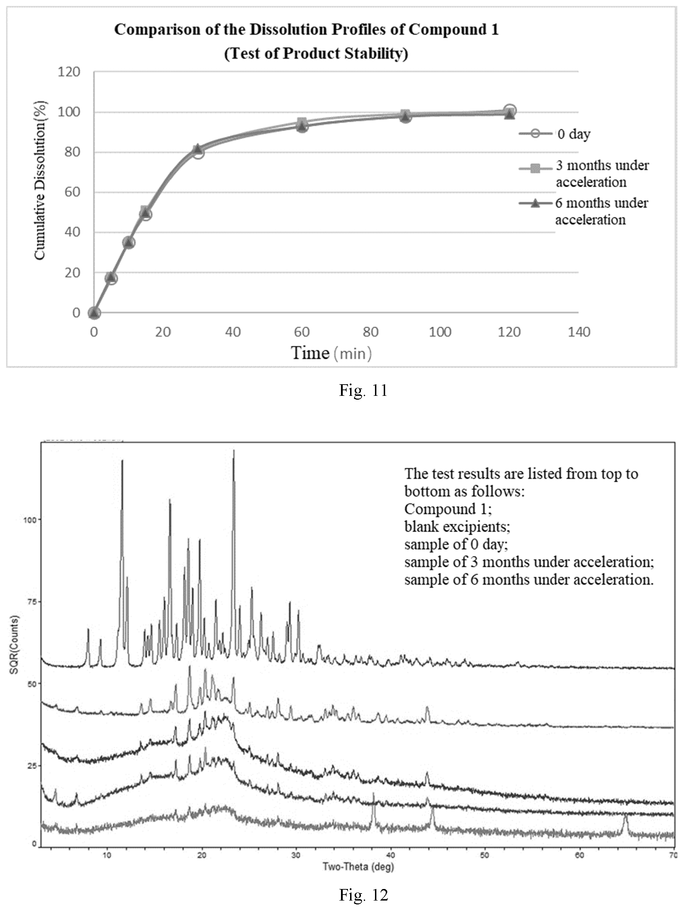

The comparison of the dissolution profile of the product for stability test is shown in Figure 11, and results of the crystal form test (XRD diffraction pattern) are shown in Figure 12.

From the above results, it can be seen that the pharmaceutical composition comprising Compound 1 according to the present invention has good stability. This property provides a good foundation for the use of such pharmaceutical compositions in the prevention and treatment of diseases, ensuring a good market application prospect for the product.

The present application has been described above in combination with preferred embodiments, but these embodiments are merely exemplary and serve an illustrative purpose omly. On this basis, various substitutions and improvements can be made to the present application, all of which fall within the protection scope of the present application.

## Claims

1. A solid dispersion, which comprises:
an active ingredient, which is 2-[3-[3-amino-4-(7H-pyrrolo[2,3-d]pyrimidin-4-yl)-1H-pyrazol-1-yl]-1-[1-[3-fluoro-2-(trifluoromethyl)isonicotinoyl]piperidin-4-yl]azetidin-3-yl] acetonitrile having a following structural formula, or a pharmaceutically acceptable salt thereof, 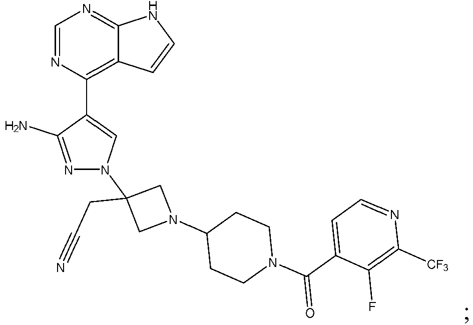
a pharmaceutically acceptable polymeric carrier; and
a surfactant;
wherein said surfactant is selected from sodium lauryl sulfate, vitamin E polyethylene glycol succinate, poloxamer, polyethylene glycol 15-hydroxystearate, sodium stearyl fumarate, sodium docusate, cetrimide, benzethonium chloride, cetylpyridinium chloride, lauric acid, polyoxyethylene alkyl ethers, polyoxyethylene sorbitan fatty acid esters; polyoxyethylene castor oil derivatives, polyoxyethylene stearates, poloxamers, polysorbates, and sorbitan fatty acid esters;
said surfactant is preferably sodium lauryl sulfate.

2. The solid dispersion according to claim 1, wherein said polymeric carrier is one or more selected from copovidone, hydroxypropyl methylcellulose phthalate (HPMCP), hydroxypropyl methylcellulose acetate succinate (HPMCAS), hydroxypropyl methylcellulose (HPMC), polymethacrylate, hydroxypropyl cellulose (HPC) and cellulose acetate phthalate (CAP); preferably, said polymeric carrier is one or more selected from copovidone, hydroxypropyl methylcellulose phthalate (HPMCP), hydroxypropyl methylcellulose acetate succinate (HPMCAS) and hydroxypropyl methylcellulose (HPMC); more preferably, said polymeric carrier is copovidone.

3. The solid dispersion according to claim 1, wherein the weight ratio of said active ingredient to said polymeric carrier is from 1:1 to 1:20, preferably from 1:2 to 1:10; and the weight ratio of said active ingredient to said surfactant is from 1:0.001 to 1:0.5.

4. A pharmaceutical composition, which comprises:
an active ingredient, which is 2-[3-[3-amino-4-(7H-pyrrolo[2,3-d]pyrimidin-4-yl)-1H-pyrazol-1-yl]-1-[1-[3-fluoro-2-(trifluoromethyl)isonicotinoyl]piperidin-4-yl]azetidin-3-yl] acetonitrile having the following structural formula, or a pharmaceutically acceptable salt thereof, 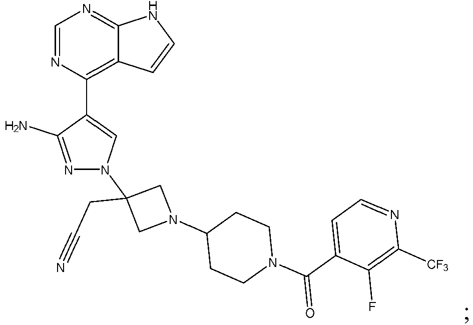
a pharmaceutically acceptable polymeric carrier;
a surfactant; and
other pharmaceutically acceptable excipients;
wherein said surfactant is selected from sodium lauryl sulfate, vitamin E polyethylene glycol succinate, poloxamer, polyethylene glycol 15-hydroxystearate, sodium stearyl fumarate, sodium docusate, cetrimide, benzethonium chloride, cetylpyridinium chloride, lauric acid, polyoxyethylene alkyl ethers, polyoxyethylene sorbitan fatty acid esters; polyoxyethylene castor oil derivatives, polyoxyethylene stearates, poloxamers, polysorbates, and sorbitan fatty acid esters;
said surfactant is preferably sodium lauryl sulfate;
wherein said active ingredient, said polymeric carrier, and optionally at least a portion of said surfactant are present in the form of a solid dispersion.

5. The pharmaceutical composition according to claim 4, wherein said polymeric carrier is one or more selected from copovidone, hydroxypropyl methylcellulose phthalate (HPMCP), hydroxypropyl methylcellulose acetate succinate (HPMCAS), hydroxypropyl methylcellulose (HPMC), polymethacrylates, hydroxypropyl cellulose (HPC) and cellulose acetate phthalate (CAP); preferably, said polymeric carrier is one or more selected from copovidone, hydroxypropyl methylcellulose phthalate (HPMCP), hydroxypropyl methylcellulose acetate succinate (HPMCAS) and hydroxypropyl methylcellulose (HPMC); more preferably, said polymeric carrier is copovidone.

6. The pharmaceutical composition according to claim 4, wherein in said solid dispersion, the weight ratio of said active ingredient to said polymeric carrier is from 1:1 to 1:20, preferably from 1:2 to 1:10; and the weight ratio of said active ingredient to said surfactant is from 1:0.001 to 1:0.5.

7. The pharmaceutical composition according to claim 4, wherein, based on the total weight of said pharmaceutical composition,
the active ingredient accounts for 0.1 wt% to 30 wt%;
the polymeric carrier accounts for 1.0 wt% to 90.0 wt%;
the surfactant accounts for 0.1 wt% to 10.0 wt%;
the total weight of other pharmaceutically acceptable excipients accounts for 10 wt% to 98 wt%.

8. The pharmaceutical composition according to claim 4, wherein the other pharmaceutically acceptable excipients include one or more of a filler, a disintegrant, a glidant, a lubricant and a binder.

9. The pharmaceutical composition according to claim 8, wherein the filler is one or more selected from lactose, sugars, starch, modified starch, mannitol, sorbitol, inorganic salts, cellulose derivatives (e.g., microcrystalline cellulose, cellulose) and xylitol; preferably, the filler is one or more selected from microcrystalline cellulose, mannitol, sorbitol and lactose.

10. The pharmaceutical composition according to claim 9, wherein the filler is microcrystalline cellulose or mannitol, or a combination of microcrystalline cellulose and mannitol;
when present, the amount of microcrystalline cellulose is from 0 wt% to 99.0 wt%, based on the total weight of said pharmaceutical composition; and/or
when present, the amount of mannitol is from 0 wt% to 99.0 wt%, based on the total weight of said pharmaceutical composition.

11. The pharmaceutical composition according to claim 8, wherein the disintegrant is one or more selected from croscarmellose sodium, crospovidone, polyvinylpyrrolidone, sodium starch glycolate, corn starch, microcrystalline cellulose, hydroxypropyl methylcellulose and hydroxypropyl cellulose; preferably, the disintegrant is one or more selected from croscarmellose sodium, crospovidone, polyvinylpyrrolidone and microcrystalline cellulose.

12. The pharmaceutical composition according to claim 8, wherein the disintegrant is croscarmellose sodium;
when present, the content of croscarmellose sodium is from 0.5 wt% to 20.0 wt%, based on the total weight of said pharmaceutical composition.

13. The pharmaceutical composition according to claim 8, wherein the glidant is selected from one or two of silicon dioxide and talc powders; preferably, the glidant is silicon dioxide;
when present, the content of silicon dioxide is from 0.1 wt% to 10.0 wt%, based on the total weight of said pharmaceutical composition.

14. The pharmaceutical composition according to claim 8, wherein the lubricant is one or more selected from magnesium stearate, magnesium lauryl stearate, sodium stearyl fumarate, stearic acid, calcium stearate, zinc stearate, potassium benzoate, sodium benzoate, myristic acid, palmitic acid, mineral oil, hydrogenated castor oil, medium-chain triglycerides, poloxamer, polyethylene glycols and talc powders; preferably, the lubricant is selected from one or two of magnesium stearate and sodium stearyl fumarate.

15. The pharmaceutical composition according to claim 8, wherein, the lubricant is magnesium stearate;
when present, the content of magnesium stearate is from 0.05 wt% to 2.0 wt%, based on the total weight of said pharmaceutical composition.

16. The pharmaceutical composition according to claim 4, wherein said pharmaceutical composition comprises the following components based on the total weight of said pharmaceutical composition:
0.1 wt% to 30.0 wt% of the active ingredient;
1.0 wt% to 90.0 wt% of copovidone;
0 wt% to 99.0 wt% of mannitol;
0 wt% to 99.0 wt% of microcrystalline cellulose;
0.5 wt% to 20.0 wt% of croscarmellose sodium;
0.1 wt% to 10.0 wt% of sodium lauryl sulfate;
0.1 wt% to 10.0 wt% of silicon dioxide;
0.05 wt% to 2.0 wt% of magnesium stearate;
wherein said active ingredient, said copovidone, and optionally at least a portion of said sodium lauryl sulfate are present in the form of a solid dispersion.

17. The pharmaceutical composition according to any one of claims 4-16, wherein said solid dispersion is prepared by spray drying process or hot-melt extrusion process; preferably, said solid dispersion is prepared by spray drying process.

18. The pharmaceutical composition according to any one of claims 4-16, wherein said pharmaceutical composition is an oral solid preparation, including a tablet, a granule, a powder, a dry suspension or a capsule.

19. The pharmaceutical composition according to any one of claims 4-16, wherein said pharmaceutical composition is a tablet.
